# EUROPÄISCHE PATENTANMELDUNG

(11) **EP 2 110 095 A1**
(43) Veröffentlichungstag der Anmeldung: **21.10.2009**
(21) Anmeldenummer: 09158152.0
(22) Anmeldetag: 17.04.2009
(51) Int. Cl.: A61B 19/02

(54) **Beutel, insbesondere zur Aufnahme von kontaminierten Gegenständen**

(30) Priorität: 18.04.2008 DE 102008019598
(71) Anmelder: Gebr. Dürrbeck Kunststoffe Gmbh, 83128 Halfing (DE)
(72) Erfinder: Dürrbeck, Joachim, 83128, Halfing (DE); Dürrbeck, Ludwig, 83128, Halfing (DE); Dürrbeck, Alfred, 99439, Buttelstedt (DE)
(74) Vertreter: Hössle Kudlek & Partner

(57) **Zusammenfassung**

Die vorliegende Erfindung betrifft einen Beutel (100, 200, 300, 400), insbesondere zur Aufnahme kontaminierter und/oder steriler Gegenstände, der wenigstens teilweise aus einem Beutelmaterial mit bioziden, biostatischen oder desinfizierenden Eigenschaften hergestellt ist, und einen Verschluss (99, 299, 399) und eine mit dem Verschluss (99, 299, 399) verschließbare Öffnung (9, 209, 309) sowie Anzeigemittel zum Anzeigen eines Wiederöffnens eines bereits verschlossenen Beutels und/oder Einmalverschlussmittel, die lediglich ein einmaliges Verschließen des Beutels gestatten, aufweist.

## Beschreibung

Die Erfindung betrifft Beutel, die insbesondere zur Aufnahme kontaminierter und/oder steriler oder zu sterilisierender Gegenstände geeignet sind.

### Stand der Technik

In der klinischen Praxis fallen täglich größere Mengen kontaminierter Gegenstände, wie etwa gebrauchtes Verbandmaterial, Tupfer und Operationsbesteck, aber auch beispielsweise Bettwäsche und Kleidung an, die entweder für eine erneute Verwendung keimfrei gemacht, oder, wenn es sich um Abfälle handelt, einer geeigneten Verwertung zugeführt werden müssen.

Infektiöse Abfälle gelangen hierbei entweder in den Sondermüll und werden dann in der Regel verbrannt, wodurch hohe Kosten entstehen; kann medizinischer Abfall hingegen durch geeignete Verfahren keimfrei gemacht werden, ist es unter Umständen möglich, diesen dann nichtinfektiösen Abfall dem Restmüll zuzugeben. Dies ist insbesondere auch für solche klinische Einrichtungen von Interesse, in denen beispielsweise aufgrund der geringen Mengen anfallenden Sterilisationsgutes oder Abfalls kein eigenes, kontinuierlich wirtschaftlich arbeitendes Abfallentsorgungs- bzw. Sterilisationssystem etabliert werden kann, wie beispielsweise dezentrale Arztpraxen oder mobile Einheiten.

Handelt es sich bei den kontaminierten Gegenständen beispielsweise um zu sterilisierendes medizinisches Gerät oder um Wäsche, ist es wünschenswert, diese Gegenstände bis zu einer geeigneten Reinigung und/oder Sterilisation sicher zu lagern bzw. so zu schützen, dass die Gegenstände sicher und ohne Kontaminationsgefahr auch durch medizinisch nicht geschultes Personal transportiert werden können.

Es besteht daher der Bedarf nach sicheren Transport-und/oder Sterilisationsbehältern für kontaminierten Abfall oder kontaminiertes medizinisches Gerät.

Ein gängiges Verfahren zur Abfallbehandlung im klinischen Bereich beinhaltet das Aussprühen von Kunststoffbeuteln mit einem geeigneten Desinfektionsmittel und das Verschweißen der Beutel mit einem Schweißgerät. Dieses Verfahren weist jedoch Nachteile auf. Der Beutel muss zunächst vollständig und gründlich ausgesprüht werden, damit das Desinfektionsmittel sich ausreichend im Beutel verteilen und seine Wirkung entfalten kann. Dies kann durch Eile oder Nachlässigkeit unvollständig erfolgen oder komplett vergessen werden. Der mit kontaminiertem Material gefüllte Beutel muss zum Schweißgerät gebracht werden, das sich häufig nicht in unmittelbarer Nähe des Füllortes befindet. Hierdurch wird möglicherweise infektiöses Material über eine nicht unbeträchtliche Distanz transportiert, was unter Umständen zur Kontamination der Umgebung führen kann. Ebenso ist denkbar, dass kontaminierte Beutel, um lästige und vermeintlich überflüssige Transportwege zu vermeiden, lange unverschlossen bleiben und eventuell durch das Personal überfüllt werden.

Das Verfahren des Aussprühens und Verschweißens birgt damit aufgrund der Vielzahl seiner Schritte und aufgrund der unpraktischen Handhabung eine Reihe möglicher Fehlerquellen, die zur Folge haben können, dass möglicherweise infektiöses Material in die Umgebung gelangt.

Sterilisationsbeutel sind bekannt. Die WO 90/04380 beschreibt einen derartigen sterilisierbaren Beutel zur Aufnahme von Gegenständen während einer Sterilisation und zum Lagern solcher Gegenstände. Der Beutel kann aus einer Schicht thermisch stabilen, vorzugsweise transparenten Kunststoffs und einer Schicht synthetischen oder natürlichen mikroorganismendichten Papiers oder aus zwei Lagen Kunststoff hergestellt sein. Der Beutel ist gemäß der WO 90/04380 mit einem Klebestreifen mit einem entfernbaren Sicherheitsband versehen, wodurch der Beutel versiegelt werden kann. Die beiden Lagen des Beutels sind voneinander trennbar (peelbar), was durch die spezielle Form der Verbindungsnaht erleichtert wird, und wodurch die sterilisierten Gegenstände einfacher entnommen werden können.

In der US 2004/0178099 A1 ist ein ähnlicher sterilisierbarer Beutel zur Aufnahme von Zahnarztspiegeln beschrieben, der zur Vermeidung von Kratzerbildung durch Aneinanderstoßen der Gegenstände im Beutel mit mehreren Fächern zur getrennten Aufnahme jeweils unterschiedlicher Gegenstände ausgestattet ist.

Die WO 99/13791 offenbart sterilisierbare Beutel, die an drei Seiten versiegelt sind und ein offenes Ende aufweisen. Ein Benutzer legt medizinische Instrumente in den Beutel ein, entfernt einen Schutzstreifen und klappt eine Lasche derart um, dass eine Klebschicht eine Kante der Beutelöffnung überlappt und hierauf angedrückt werden kann.

Falls in den vorgenannten Schriften eine Keimfreimachung des Beutelinhaltes überhaupt vorgesehen ist, wird hierzu durchweg ein Sterilisationsgerät benötigt.

Mit bioziden bzw. biostatischen Mitteln versehene Kunststoffe, hieraus hergestellte Folien und betreffende Behälter sind ebenfalls bekannt. Die JP 03272764 A beschreibt beispielsweise einen Beutel zur Aufnahme medizinischen Abfalls, der aus einfachen oder zusammengesetzten, antibakteriellen Polyolefinfolien hergestellt ist. Der Beutel wird nach Einlegen des infektiösen Materials evakuiert und mit einem Desinfektionsmittel versehen. Anschließend wird der Beutel verschweisst.

Aufgabe der Erfindung ist die Bereitstellung eines in seiner Herstellung und/oder Wirksamkeit gegenüber dem Stand der Technik verbesserten Beutels.

### Vorteile der Erfindung

Die Aufgabe wird gelöst durch einen Beutel mit den Merkmalen des Patentanspruchs 1.

Der erfindungsgemäße Beutel besteht aus einem geeigneten Beutelmaterial mit bioziden, biostatischen oder desinfizierenden Eigenschaften, und weist einen Verschluss auf, mittels dessen eine Öffnung des Beutels verschließbar ist. Somit ist es durch die Erfindung in besonders vorteilhafter Weise möglich, den Beutel vor Ort zu verschließen, da der Verschluss am Beutel selbst angebracht ist. Wie bereits oben diskutiert, sind zur Abfallbehandlung im klinischen Bereich bisher vornehmlich verschweißbare Behälter vorgesehen. Beutel mit entsprechender biozider, biostatischer oder desinfizierender Ausstattung und mit einem am Beutel selbst vorgesehenen Verschluss sind hingegen nicht bekannt. Die Beutel müssen daher herkömmlicherweise stets zu einem geeigneten Schweißgerät transportiert werden, welches sich häufig nicht in unmittelbarer Nähe der Befüllungsstelle befindet. Die hierdurch bestehenden Nachteile, verbunden mit den genannten zusätzlichen Problemen wie einer möglichen Kontamination der Umgebung mit infektiösem Material, werden durch die vorliegende Erfindung überwunden. Ein entsprechender Beutel ist also autark handhabbar und bedarf zu seinem ordnungsgemäßen Verschluss keiner weiteren Vorrichtungen. Wie bereits oben genannt, können entsprechende Beutel insbesondere in mobilen medizinischen Einrichtungen (beispielsweise in Krankenwagen) zum Einsatz kommen, wo dieses erfindungsgemäße Merkmal von besonderem Vorteil ist.

Der Beutel weist ferner Anzeigemittel zum Anzeigen eines Wiederöffnens eines bereits verschlossenen Beutels und/oder Einmalverschlussmittel, die lediglich ein einmaliges Verschließen des Beutels gestatten, auf. Hierdurch wird bewirkt, dass beispielsweise eine Wiederverwendung eines zum Einmalgebrauch bestimmten Beutels vermieden wird und gewährleistet ist, dass kein kontaminiertes Material unbemerkt aus dem Beutel entnommen oder dem Beutel zugegeben werden kann. Die bioziden, biostatischen und/oder desinfizierenden Eigenschaften des Beutels können beispielsweise dadurch bewirkt werden, dass dem Beutelmaterial ein biozides, biostatisches und/oder desinfizierendes Material, etwa in Form eines Granulates, zuzugeben wird. Hierdurch kann der Beutel direkt aus dem dann bioziden bzw. biostatischen Beutelmaterial hergestellt werden. Nachfolgende Ausrüstungs- oder Oberflächenbeschichtungsschritte können dann entfallen, was eine bedeutend kostengünstigere Herstellung oder erleichterte Handhabung ermöglicht. Ein Beispiel für ein Biozid-Additiv, das in einer Dosierung von beispielsweise 1 bis 5 % dem Kunststoff zugegeben wird und im Großmaßstab einsetzbar ist, ist das Additiv BZ030-PE der Firma GRAFE Advanced Polymers GmbH, Blankenhain. Andere Additive, die verwendet werden können, beinhalten beispielsweise Silberpartikel. Es sind auch flüchtige Additive verwendbar, die, wenn sie dem Kunststoff beigegeben werden, über einen bestimmten Zeitraum in die Gasphase übergehen (slow release). Alternativ dazu kann das Beutelmaterial auf klassische Weise mit dem biozid, biostatisch und/oder desinfizierend wirkenden Material oder Agens beschichtet oder anderweitig ausgestattet werden

Bevorzugte Ausgestaltungen des erfindungsgemäßen Beutels sind Gegenstand der abhängigen Ansprüche.

Gemäß einer besonders bevorzugten Ausführungsform des erfindungsgemäßen Beutels weist der Verschluss einen Klebestreifen auf, der mit einem abziehbaren Sicherheitsstreifen ausgebildet ist. Der Sicherheitsstreifen wird vor den Zukleben des Beutels abgezogen und schützt zuvor die Klebefläche. Aus unterschiedlichen Gründen ist eine derartige Ausgestaltung des Beutels besonders vorteilhaft: Der Schutz des Klebestreifens durch einen Sicherheitsstreifen bewirkt, dass der im Klebestreifen enthaltene Klebstoff bis zur Verwendung weitestgehend von den Lagerungsbedingungen (ggf. Trockenheit, Staub) unabhängig seine optimale Haftkraft bewahrt und damit den oben dargestellten erfindungsgemäßen bakteriendichten Verschluss des Beutels garantiert. Besonders im Falle größerer Beutel besteht bei einem freiliegenden Klebestreifen und der einhändigen Handhabung des Beutels (die andere Hand des Benutzers legt ggf. kontaminiertes Material in den Beutel ein) zudem die Gefahr, dass der Klebestreifen des Beutels mit der Kleidung des Benutzers oder mit Schutzhandschuhen in Kontakt kommt und daran festklebt, woraufhin der Klebestreifen wieder abgezogen werden muss, ggf. hierdurch an Haftkraft einbüßt und unbrauchbar wird. Ein weiteres Problem, das durch das Bedecken einer freiliegenden Klebefläche durch einen Sicherheitsstreifen vermieden wird, ist das Aneinanderkleben von Bereichen der Klebefläche, wodurch etwa Falten und Luftkanäle entstehen könnten, die ein nachfolgendes bakteriendichtes Zukleben des Beutels verhindern. Es wird weiterhin durch den Sicherheitsstreifen vermieden, dass Füllgut am Klebestreifen festklebt, was ebenfalls zu den oben beschriebenden Problemen führen kann. Die Lagerung wird durch einen mit einem Sicherheitsstreifen bedeckten Klebestreifen bedeutend erleichtert.

Zweckmäßigerweise sind die Anzeigemittel und die Einmalverschlussmittel durch den Verschluss bereitgestellt. Wenn ein solcher Verschluss Mittel bereitstellt, die bewirken, dass der Wiederverschluss durch verminderte Haftkraft des Klebestreifens nach einer zuvor erfolgten Öffnung verhindert wird und/oder das Wiederöffnen durch eine Konsistenz-und/oder Farbveränderung des Verschlusses angezeigt wird und/oder das Wiederöffnen ein Beschädigen des Verschlusses bewirkt, wird hierdurch ein Beutel bereitgestellt, mit dem verhindert wird, dass später unkontrolliert Material entnommen oder zugegeben wird, oder ein wegwerfbarer Beutel unerlaubterweise erneut verwendet wird, bei welchem dann beispielsweise die Biozidwirkung erschöpft ist. Vorteilhafterweise kann der Wiederverschluss etwa durch eine verminderte Haftkraft oder eine Konsistenzveränderung des Klebstoffes nach dem Aufreissen des Beutels verhindert werden, oder dadurch, dass der Verschluss, bewirkt etwa durch Sollrissstellen oder durch eine besonders intensive Haftkraft des Klebstoffs, durch die Wiederöffnung zwangsläufig beschädigt wird. Diese Beschädigung dient dann gleichzeitig dazu, das Öffnen des Beutels anzuzeigen. Alternativ kann dies erreicht werden, indem ein Öffnungsindikator verwendet wird, der beispielsweise in einem Klebstoffstreifen besteht, dem ein zug- bzw. drucksensitiver Farbstoffindikator zugegeben wird, und der bei einem versuchten Öffnen mit einer Farbveränderung reagiert.

Gemäß einer besonders bevorzugten Ausführungsform, um die gesondert um Schutz nachgesucht wird, kann ein Beutelmaterial wenigstens zwei Schichten aufweisen, von denen nur eine, insbesondere die dem Beutelinnenraum zuweisende Schicht, biozide, biostatische und/oder desinfizierende Eigenschaften aufweisen kann. Neben der Zugabe des Biozids zum gesamten Beutelmaterial kann es nämlich auch vorteilhaft sein, nur einen Teil bzw. eine Schicht des Beutelmaterials mit dem Biozid zu versehen, da hierdurch spezielle, den jeweiligen Erfordernissen angepasste Beuteleigenschaften erzielt werden können.

So kann es beispielsweise von Vorteil sein, die innerste Schicht des Beutels biozid, die äußerste Schicht hingegen biostatisch zu gestalten. Birgt beispielsweise ein verwendetes Biozid für den Benutzer bestimmte Gefahren (Hautreizung, Allergieauslösung, toxische Wirkung usw.), so kann dieses nur an der Beutelinnenseite oder nur in einer oder auf einer inneren Schicht vorgesehen sein. Die in direktem Kontakt mit dem Benutzer stehende Beutelaussenseite (äußere Schicht) kann hingegen eine gesundheitlich unbedenkliche Substanz aufweisen und trotzdem eine weitere Ausbreitung unbeabsichtigt auf die Beutelaußenseite gelangter Keime bremsen, oder aber vollkommen wirkstofffrei sein. Weiterhin kann beispielsweise für die innerste Schicht ein flüchtiges Biozid verwendet werden, das sich im Beutelinneren in der Gasphase ausbreitet, anreichert, und damit eine Sterilisation des Inhalts bewirkt, wohingegen an der äußeren Beutelfläche ein nichtflüchtiges (Kontakt-) Biozid oder Biostatikum vorgesehen sein kann.

Das Beutelmaterial kann vorteilhafterweise einen Farbstoff und/oder Sichtschutzmittel aufweisen. Es kann sich als vorteilhaft erweisen, den Beutel beispielsweise mit einer spezifischen Farbe auszustatten, wodurch auf das im Beutel enthaltene Material hingewiesen oder vor dem Öffnen des Beutels gewarnt wird. Weiterhin kann hierdurch ein Farbschema für bestimmte Arten im jeweiligen Beutel transportierten Materials realisiert oder eine Zeitzuordnung vorgenommen werden. Wird in dem Beutel stark verschmutztes, unansehnliches oder ekelerregendes Material transportiert, kann ein Sichtschutz durch eine nichttransparente Einfärbung des Beutelmaterials von Vorteil sein, insbesondere, wenn solche Beutel sich beispielsweise in durch Besucher oder Patienten einsehbaren Bereichen befinden.

Der Beutel wird vorteilhafterweise aus zwei Lagen Material, vorzugsweise aus Kunststoff, insbesondere aus Polyethylen hergestellt, die an drei Kanten durch wenigstens eine Seitennaht miteinander verbunden sind, wodurch eine Beuteloberseite, eine Beutelunterseite und der Beutelinnenraum definiert ist. Die Seitennaht kann als Schrumpf- oder Doppelschrumpfnaht ausgeführt sein. Dem Fachmann sind geeignete Kunststoffe zur Herstellung von Beuteln bekannt; vorzugsweise wird hierzu Polyethylen verwendet, das als LDPE, LLDPE, MDDPE, MMDPE oder HDPE mit unterschiedlicher, jeweils dem Einsatzzweck entsprechender Vernetzung und Permeabilität verwendet werden kann. Der Vorteil der Herstellung aus Kunststofffolie, insbesondere aus Polyethylen, liegt in der relativen chemischen Inertheit der Polyolefine gegenüber in Frage kommenden Chemikalien und die einfache, routinemäßige Bearbeitbarkeit betreffender Kunststoffe durch gängige Verfahren.

Es vorteilhaft sein, dass die vierte Kante wenigstens teilweise mit dem Verschluss ausgebildet ist, oder dass der Verschluss auf der Beutelober- oder Unterseite ausgebildet ist. Den Verschluss an der vierten Beutelkante auszubilden kann hierbei vorteilhaft sein, wenn etwa erwünscht ist, einen Verschluss in Form einer Verschlusslasche, die aus einer verlängerten Beutelseite hergestellt ist, bereitzustellen. Beispielsweise kann durch die Bereitstellung an einer der Kanten eine besondere Einreißfestigkeit sehr einfach dadurch erzielt werden, dass die Seitennaht in Nähe der Öffnung verstärkt wird, wohingegen die Ausbildung auf einer Beutelseite Vorteile beim Einführen bestimmter, beispielsweise flacher Gegenstände in den Beutel erbringen kann, während dieser flach auf einer Oberfläche aufliegt. Im Falle einer Öffnung auf der Vorderseite kann ein Beutel beispielsweise auch, wie weiter unten dargestellt, an einer Aufhängelochung aufgehängt sein und im aufgehängten Zustand befüllt werden.

Der Verschluss des Beutels kann vorteilhafterweise durch Umklappen und Anheften einer Verschlusslasche mit dem Klebestreifen erfolgen. Wird beispielsweise flaches, rechteckiges Material in den Beutel eingeführt, kann es sich als nützlich erweisen, eine solche Verschlussklappe vorzusehen, da jene entlang einer Kante des sich im Beutel befindenden Materials umgeklappt und auf dem Material angedrückt werden kann.

Es kann jedoch auch vorteilhaft sein, einen Klebestreifen entlang der Öffnung an einer Kante auf der dem Beutelinnenraum zuweisenden Seite einer Lage des Beutelmaterials auszubilden, wobei der Beutel durch Verkleben des Klebestreifens mit der gegenüberliegenden, dem Beutelinnenraum zuweisenden Seite der anderen Lage des Beutelmaterials verschließbar ist. Wird ein Beutel im aufrechten Zustand befüllt, und enthält das kontaminierte Material etwa Flüssigkeiten, die beim Kippen des Beutels auslaufen könnten, kann es sich als nützlich erweisen, den Beutel durch das hierbei mögliche Aneinanderdrücken der offenen Beutelkanten zu verschließen. Ein Druckleistenverschluss (Zip Lock) kann hierbei von besonderem Vorteil sein, da dieser erlaubt, einen Beutel zunächst vorläufig zu verschließen, wodurch die Verschlusskanten in parallele Anordnung gebracht werden, und danach den Beutel endgültig und dicht zu verkleben. Wenn ein Beutel mit voluminösem Material gefüllt ist, das ein Aufwölben der Beutelflächen bewirkt, kann es hingegen auch zweckmäßig sein, einen Beutel mit einem separaten Verschlussklebestreifen bereitzustellen, der über einen Öffnungsschlitz geklebt wird, da sich beispielsweise das Umklappen einer Lasche hier als schwierig erweisen könnte.

Ein durchstichsicher und/oder schnittsicher ausgeführter Beutel ist zur Aufnahme scharfer und/oder spitzer Gegenstände wie etwa von Skalpellen, Kanülen oder Glasbruch besonders geeignet, da hierbei eine Verletzung des Benutzers mit kontaminiertem Material vermieden werden kann. Dies kann beispielsweise durch eine Gewebeeinlage im Beutelmaterial oder durch eine entsprechende Materialstärke erreicht werden.

Im Falle einer nicht vermeidbaren Beschädigung des Beutels ist es besonders vorteilhaft, zumindest ein Auslaufen flüssigen Beutelinhaltes zu vermeiden. Hierzu kann ein Absorbermaterial für Flüssigkeiten im Beutel vorgesehen sein, welches entweder lose im Beutel angeordnet oder in geeigneter Weise auf die Beutelinnenseite aufgebracht ist, wobei entweder ein Absorbermaterial dem Beutelmaterial zugegeben wird oder hierauf appliziert ist. Geeignete Flüssigkeitsabsorber sind dem Fachmann bekannt; vorzugsweise können etwa Natrium-Polyacrylate verwendet werden, die zudem mit dem gleichen oder einem anderen bioziden oder biostatischen Material wie im Beutel selbst versetzt sein können.

Falls erforderlich, etwa wenn das kontaminierte Material ausgasende Substanzen enthält, oder wenn das Biozid oder das Biostatikum in die Gasphase im Beutelinneren übertritt, oder wenn die Krankheitserreger oder Schädlinge in dem in den Beutel eingebrachten Material Sauerstoff zu ihrer Vermehrung benötigen, kann es besonders vorteilhaft sein, wenn der Beutel nach dem Verschließen gasdicht abgeschlossen ist. Wenn geeignete Mittel, wie etwa ein Absaugventil bereitgestellt sind, ist es dann in besonders vorteilhafter Weise zusätzlich möglich, den Beutel zu evakuieren. Hierdurch kann beispielsweise das Stauvolumen gefüllter Beutel minimiert oder der verfügbare Sauerstoff im Beutel auf ein Minimum beschränkt werden.

Für eine optimale Handhabung ist es nützlich, eine Beutelhalterung vorzusehen, in die mit einer Aufhängelochung versehene Beutel eingehängt werden können. Die Beutel können dann von der Aufhängevorrichtung abgenommen oder an einer ggf. ebenfalls vorgesehenen Aprissperforation von der Halterung abgerissen werden. Hierdurch kann ein geeigneter Beutelspender zum Einsatz kommen, der einen optimalen Arbeitsablauf ermöglicht.

Zur lückenlosen Dokumentation können bei einer Abrissperforation der Abrissrest und der Beutel selbst identische Identifizierungsmerkmale, wie etwa eine laufende Nummer oder einen Barcode enthalten, so dass der Verbleib jedes Beutels nachgewiesen und dokumentiert werden kann.

### Zeichnung

Weitere Einzelheiten und Vorteile der Erfindung gehen aus der nachfolgenden Figurenbeschreibung hervor, in welcher mehrere bevorzugte Ausführungsformen rein beispielhaft, jedoch in keiner Weise den Umfang der Erfindung einschränkend dargestellt sind.

Die Zeichnung, die Beschreibung und die Ansprüche enthalten zahlreiche Merkmale in Kombination; der Fachmann wird diese und weitere Merkmale dabei zweckmäßigerweise selbstverständlich auch einzeln betrachten und/oder zu sinnvollen weiteren Kombinationen zusammenfassen.

Es zeigen in der Zeichnung:
Fig. 1 eine Draufsicht einer ersten bevorzugten Ausführungsform eines erfindungsgemäßen Beutels,
Fig. 2 eine Draufsicht einer zweiten bevorzugten Ausführungsform eines erfindungsgemäßen Beutels,
Fig. 3 eine dreidimensionale Ansicht einer dritten bevorzugten Ausführungsform eines erfindungsgemäßen Beutels, und
Fig. 4 eine Draufsicht einer Teilansicht einer vierten bevorzugten Ausführungsform eines erfindungsgemäßen Beutels;
wobei gleiche Bezugszeichen gleiche oder entsprechende Elemente bezeichnen und auf eine wiederholte Beschreibung nachfolgend der Einfachheit halber verzichtet wird, sofern die entsprechenden Elemente gleichartig oder hinreichend ähnlich ausgebildet sind.

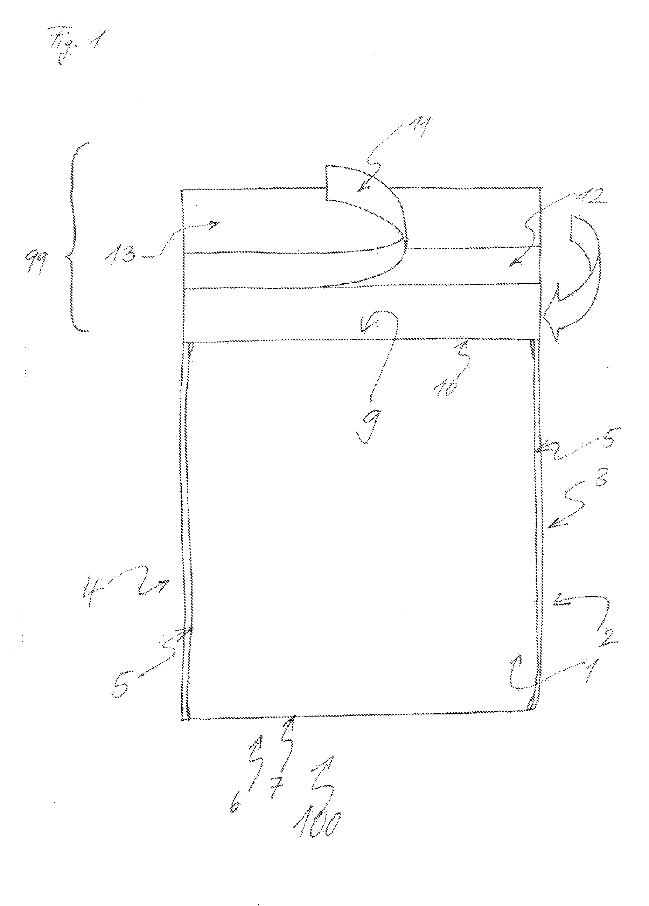

In Figur 1 ist eine erste bevorzugte Ausführungsform eines erfindungsgemäßen Beutels in einer schematisch vereinfachten Draufsicht dargestellt.

Der in seiner Gesamtheit mit 100 bezeichnete Beutel weist zwei Lagen Beutelmaterial 1, 2 aus beispielsweise Kunststoff, vorzugsweise aus Polyethylen auf, durch welche eine Beutelober- und eine Beutelunterseite sowie ein Beutelinnenraum definiert wird. Die Beutelmateriallagen 1, 2 können dabei selbst ein- oder mehrschichtig ausgestaltet sein. Jede Schicht des Beutelmaterials 1, 2 kann hierbei mit einem Biozid, einem Biostatikum oder einem Desinfektionsmittel versehen sein, das dem Material zugegeben oder hierauf aufgebracht ist. Bei einschichtiger Ausgestaltung des Beutelmaterials kann das Biozid hierbei dem gesamten Material zugegeben sein, etwa in Form eines Granulats, bei mehrschichtiger Ausgestaltung können die einzelnen Schichten jeweils unterschiedliche biozide, biostatische, desinfizierende oder keine biologisch aktiven Eigenschaften aufweisen. Die derart geschaffenen Materiallagen 1, 2 sind an zwei Kanten 3, 4 randständig mit einer Seitennaht 5 verschlossen, wobei in der Zeichnung die Beutelunterkante 6 durch einen Materialfalz 7 gebildet wird. Die Seitennaht 5 ist dabei vorzugsweise als Schrumpf- oder Doppelschrumpfnaht ausgebildet. Alternativ hierzu, aber hier nicht zusätzlich dargestellt, kann die Beutelunterkante 6 ebenso als eine entsprechende Seitennaht in analoger Ausführung ausgebildet sein. Auch können die übereinander liegenden Lagen Beutelmaterial 1, 2 an der Beutelunterkante 6 beispielsweise einen Doppelfalz aufweisen, um das nutzbare Beutelvolumen zu vergrößern. Der Zugang zum Beutelinnenraum, welcher durch die zwei Lagen Beutelmaterial 1,2 und die randständige Seitennaht 5 definiert wird, erfolgt über die Öffnung 9, die hier durch die Kante 10 der Beuteloberseite 1 definiert wird, jedoch auch, falls beispielsweise zwei Lagen Beutelmaterial 1, 2 an vier Seiten auf geeignete Weise verschlossen sind, als Schlitz in einer entsprechenden Lage 1 oder 2 des Beutelmaterials ausgebildet sein kann.

Der erfindungsgemäße Beutel der ersten Ausführungsform weist einen Sicherheitsstreifen 11 auf, welcher in der Abbildung in teilweise abgezogenem Zustand dargestellt ist, und welcher bei Nichtgebrauch, aber auch bei der Befüllung des Beutels 100 mit beispielsweise kontaminiertem Material einen darunterliegenden, zum Verschließen des Beutels dienenden, und gegebenenfalls weitere Merkmale aufweisenden Klebestreifen 12 bedeckt, um jenen in der weiter oben dargestellten Weise vor schädlichen Einflüssen (Trockenheit, Staub etc.) während der Lagerung, vor Berührung durch die Befüllungsperson, vor dem Kontakt mit Befüllungsgut, und/oder vor dem Aneinanderkleben zu schützen.

Der mit dem Sicherheitsstreifen 11 bedeckte Klebestreifen 12 befindet sich auf einer durch einen Abschnitt des Beutelmaterials definierten Lasche 13, und kann nach Abziehen des Sicherheitsstreifens 11 zum Verschließen des Beutels 100 nach unten der Pfeilrichtung entsprechend auf die Öffnung 9 umgeklappt und hierauf manuell angedrückt werden, um einen ordnungsgemäßen dichten Verschluss des Beutels 100 sicherzustellen, und zu verhindern, dass in den Beutel 100 eingelegtes kontaminiertes Material oder in oder auf diesem befindliche Kontaminanten nach außen gelangen können. Der Sicherheitsstreifen 11 und der Klebestreifen 12, angeordnet auf Lasche 13 bilden in ihrer Gesamtheit damit einen Verschluss 99.

Der durch die Lasche 13, den Sicherheitsstreifen 11 und den Klebestreifen 12 definierte Verschluss 99 kann zusätzlich Mittel zum Anzeigen eines Wiederöffnens eines bereits verschlossenen Beutels und/oder Mittel, die lediglich ein einmaliges Verschließen des Beutels 100 gestatten, aufweisen. Beispielsweise kann etwa eine nach dem erstmaligen Verschließen verminderte Haftkraft des Klebestreifens 12, eine durch eine versuchte Öffnung des Verschlusses 99 bewirkte Beschädigung der Lasche 13 und/oder eine durch eine versuchte Öffnung des Verschlusses 99 und durch einen Indikator bewirkte Farbveränderung vorgesehen sein.

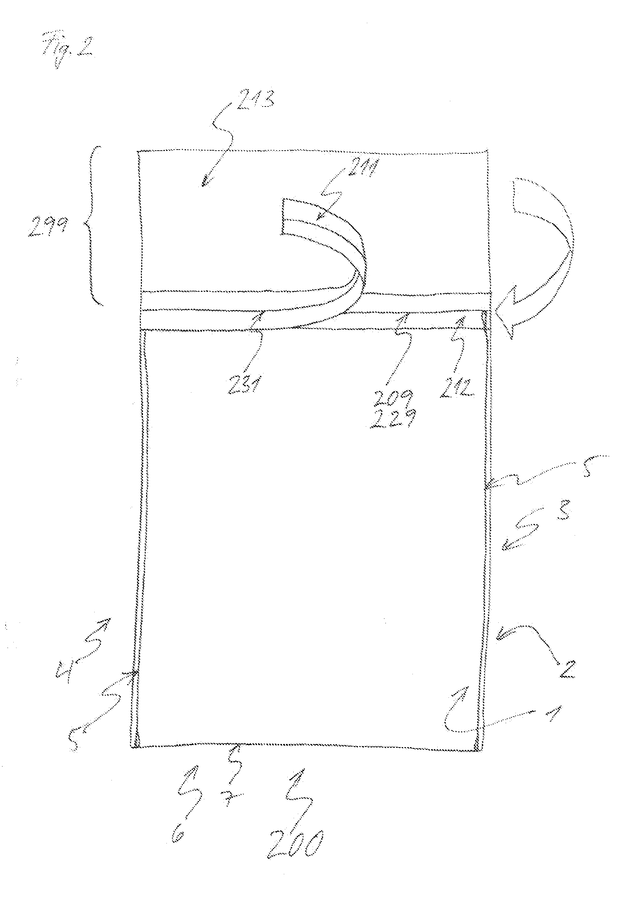

In Figur 2 ist eine zweite bevorzugte Ausführungsform eines erfindungsgemäßen Beutels in einer schematisch vereinfachten Draufsicht dargestellt, der in seiner Gesamtheit mit 200 bezeichnet ist.

Die zunächst im wesentlichen ähnlich jener der ersten bevorzugten Ausführungsform ausgebildete Öffnung 209 ist entsprechend der Ausgestaltung dieser Ausführungsform jedoch vollständig von Klebstoff in Form eines Klebestreifens 212 umgeben. Der Klebestreifen 212 weist einen Schlitz 229 auf, der entsprechend der Öffnung 209 dimensioniert ist, und hiermit einen Durchgriff durch den Klebestreifen 229 erlaubt. Zusätzlich, aber in der Zeichnung nicht weiter dargestellt, kann der Klebestreifen 229 von der Beutelöffnung 209 zweckmäßigerweise auch zusätzlich durch einen ausreichend breiten klebstofffreien Sicherheitsabstand beabstandet sein, wodurch verhindert wird, dass der Benutzer, oder vom Benutzer in den Beutel eingelegtes kontaminiertes Material mit der Kante des Klebestreifens 229 in Kontakt kommt.

Auch im Rahmen dieser bevorzugten Ausführungsform ist der Klebestreifen 212 durch einen Sicherheitsstreifen 211 bedeckt, der hier jedoch zusätzlich mit einem Schlitz 231 in gleicher Dimensionierung wie der darunterliegende Schlitz 229 im Klebestreifen 212 und in gleicher Dimensionierung wie die Öffnung 209 versehen ist, wodurch eine Befüllung des Beutels 200 hierdurch erlaubt wird. Nach dem Befüllen des Beutels wird der Sicherheitsstreifen 211 auch hier abgezogen, und es kann eine klebstoffrei ausgebildete Lasche 213, die hier durch einen Abschnitt der Lage 2 des Beutelmaterials definiert ist, dem Pfeil entsprechend auf den freigelegten Klebstoffstreifen 212 umgeklappt und hierauf angedrückt werden.

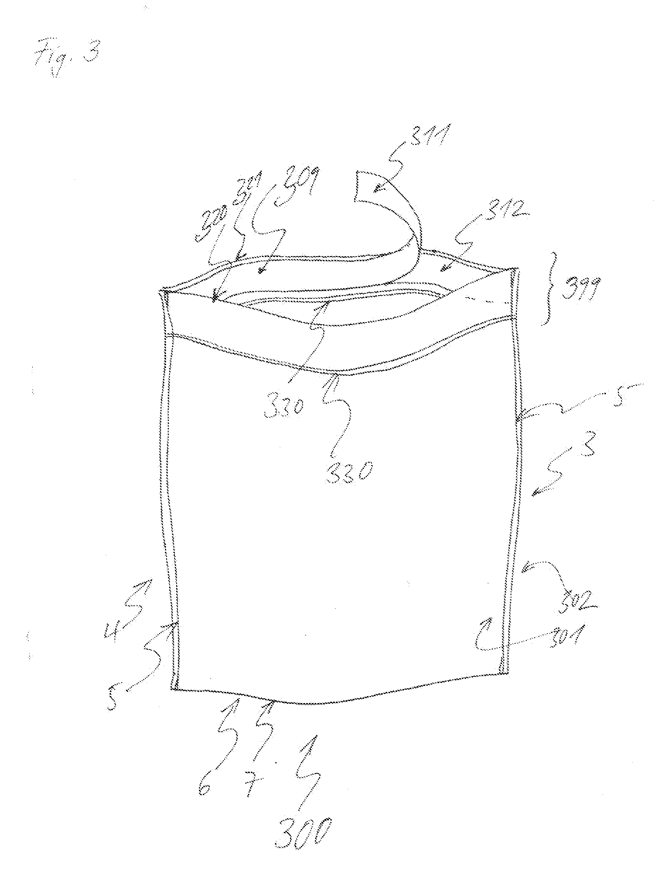

In Figur 3 ist eine dritte bevorzugte Ausführungsform eines erfindungsgemäßen Beutels in einer schematisch vereinfachten teilperspektivischen Ansicht dargestellt, wobei der Beutel insgesamt mit 300 bezeichnet und in teilweise geöffnetem Zustand abgebildet ist.

Auch der Beutel dieser Ausführungsform weist zwei Lagen Beutelmaterial 301, 302 geeigneter Materialcharakteristik auf, die, wie entsprechend oben im Rahmen der Beschreibung zu Fig. 1 dargestellt, an den Kanten 303, 304, 305 aneinandergefügt sind, wodurch ein Beutelinnenraum definiert ist. Im Rahmen der hier diskutierten Ausführungsform weisen die Beutelober- und die Beutelunterseite 301, 302 jedoch eine im wesentlichen gleiche Größe auf. Die vierten Kanten 320, 321 der Beutelmateriallagen 301, 302 sind zunächst nicht miteinander verbunden und definieren hierdurch eie Öffnung 309.

Entlang der Öffnung 309 ist an der Kante 321 auf der dem Beutelinnenraum zuweisenden Seite der Beutelmateriallage 2 ein Klebestreifen 312 ausgebildet, der durch einen Sicherheitsstreifen 311 bedeckt ist. Nach dem Befüllen des Beutels 300 wird der Klebestreifen 312 abgezogen und ein Verschluss des Beutels durch manuelles Aneinanderdrücken des Klebestreifens 312 und der dem Beutelinnenraum zuweisenden Seite der Beutelmateriallage 1 an der Kante 320 bewirkt.

Zusätzlich ist in der Figur ein Druckleistenverschluss (Zip Lock) 330 dargestellt, der nicht notwendigerweise zum Verschluss des Beutels erforderlich ist, da dieser durch den Klebestreifen 312 bewirkt wird. Er kann jedoch, beispielsweise bei der Befüllung des Beutels 300 mit voluminösem Material, von Vorteil sein, da hierdurch die Beutelkanten 320, 321 in parallele Anordnung gebracht werden können, und der Beutel hiernach in einfacher Weise endgültig verklebt wird.

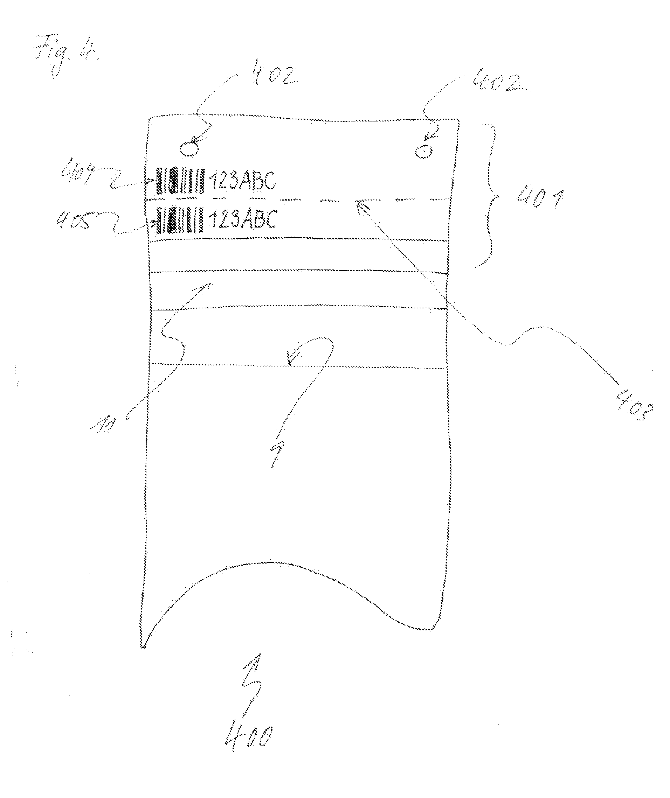

Figur 4 zeigt die Teilansicht der Oberseite einer vierten Ausführungsform eines erfindungsgemäßen Beutels, der insgesamt mit dem Bezugszeichen 400 bezeichnet ist.

Die Ausgestaltung des Beutels 400 selbst entspricht weitgehend jener der vorstehend beschriebenen Ausführungsformen. Zusätzlich ist hier jedoch eine Lasche 401 vorgesehen, die verlängert ist und eine Aufhängelochung 402 aufweist, mit Hilfe derer der Beutel beispielsweise an einer Beutelhalterung, die in der Zeichnung nicht dargestellt ist, aufgehängt werden kann. Zusätzlich ist eine Abrissperforation 403 vorgesehen, entlang derer der Beutel beispielsweise von einer Halterung, an die er mit der Aufhängelochung 402 angebracht ist, abgerissen werden kann.

Weiter weist der Beutel gemäß Ausführungsform beiderseits der Abrissperforation angebrachte Identifizierungsmerkmale 404, 405, die hier in beispielhafter Darstellung durch eine alphanumerische Zeichenfolge und einen Barcode dargestellt sind, auf. Vorzugsweise sind diese Identifizierungsmerkmale identisch und erlauben eine Zuordnung eines beispielsweise an der Beutelhalterung verbleibenden Abrissrests zu einem jeweils hiervon abgerissenen Beutel und damit eine lückenlose Dokumentation der Verwendung der Beutel.

## Patentansprüche

1. Beutel (100, 200, 300, 400), insbesondere zur Aufnahme kontaminierter und/oder steriler Gegenstände, **dadurch gekennzeichnet, dass** er wenigstens teilweise aus einem Beutelmaterial mit bioziden, biostatischen oder desinfizierenden Eigenschaften hergestellt ist, und einen Verschluss (99, 299, 399) und eine mit dem Verschluss (99, 299, 399) verschließbare Öffnung (9, 209, 309) sowie Anzeigemittel zum Anzeigen eines Wiederöffnens eines bereits verschlossenen Beutels und/oder Einmalverschlussmittel, die lediglich ein einmaliges Verschließen des Beutels gestatten, aufweist.

2. Beutel (100, 200, 300, 400) nach Anspruch 1, **dadurch gekennzeichnet, dass** der Verschluss (99, 299, 399) einen Klebestreifen (12, 212, 312) aufweist, der mit einem abziehbaren Sicherheitsstreifen (11, 211, 311) ausgebildet ist.

3. Beutel (100, 200, 300, 400) nach Anspruch 1 oder 2, **dadurch gekennzeichnet, dass** die Anzeigemittel und/oder die Einmalverschlussmittel durch den Verschluss (99, 299, 399) bereitgestellt werden, wobei der Verschluss (99, 299, 399) Mittel aufweist, die bewirken, dass der Wiederverschluss durch verminderte Haftkraft des Klebestreifens (12, 212, 312) nach einer zuvor erfolgten Öffnung verhindert wird und/oder das Wiederöffnen durch eine Konsistenz- und/oder Farbveränderung des Verschlusses (99, 299, 399) angezeigt wird und/oder das Wiederöffnen ein Beschädigen des Verschlusses (99, 299, 399) bewirkt.

4. Beutel (100, 200, 300, 400) nach einem der vorstehenden Ansprüche oder dem Oberbegriff von Anspruch 1, **dadurch gekennzeichnet, dass** das Beutelmaterial wenigstens zwei Schichten aufweist, wobei wenigstens eine, insbesondere die einem Beutelinnenraum zuweisende Schicht, biozide, biostatische und/oder desinfizierende Eigenschaften aufweist.

5. Beutel (100, 200, 300, 400) nach Anspruch 4, **dadurch gekennzeichnet, dass** die dem Beutelinnenraum zuweisende Schicht biozide oder desinfizierende Eigenschaften, und die nach außen weisende Schicht biostatische Eigenschaften oder keine bioziden, biostatischen oder desinfizierenden Eigenschaften aufweist.

6. Beutel (100, 200, 300, 400) nach einem der vorstehenden Ansprüche, **dadurch gekennzeichnet, dass** das Beutelmaterial einen Farbstoff und/oder Sichtschutzmittel aufweist.

7. Beutel (100, 200, 300, 400) nach einem der vorstehenden Ansprüche, **dadurch gekennzeichnet, dass** der Beutel wenigstens zwei Lagen übereinander angeordneten folienartigen Beutelmaterials (1, 2, 301, 302), vorzugsweise aus Kunststoff, insbesondere aus Polyethylen, aufweist, die an wenigstens drei Kanten (3, 4, 6) miteinander verbunden sind, wodurch eine Beuteloberseite (1, 301), eine Beutelunterseite (2, 302) und der Beutelinnenraum definiert ist.

8. Beutel (100, 200, 300, 400) nach Anspruch 7, **dadurch gekennzeichnet, dass** die vierte Kante wenigstens teilweise mit dem Verschluss (99, 299, 399) ausgebildet ist oder der Verschluss (99, 299) auf der Beutelober- oder Unterseite ausgebildet ist.

9. Beutel (100, 200, 300, 400) nach einem der Ansprüche 2 bis 8, **dadurch gekennzeichnet, dass** das Verschließen des Beutels durch Umklappen und Anheften einer Verschlusslasche (13, 213) mit dem Klebestreifen (12, 212) erfolgt.

10. Beutel (100, 200, 300, 400) nach einem der Ansprüche 2 bis 9, **dadurch gekennzeichnet, dass** der Klebestreifen (312) entlang der Öffnung an einer Kante (321) auf der dem Beutelinnenraum zuweisenden Seite einer Lage (302) des Beutelmaterials ausgebildet ist und der Verschluss durch Anheften der gegenüberliegenden, dem Beutelinnenraum zuweisenden Seite der anderen Lage (301) des Beutelmaterials erfolgt.

11. Beutel (100, 200, 300, 400) nach einem der vorstehenden Ansprüche, **dadurch gekennzeichnet, dass** der Beutel ganz oder teilweise durchstichsicher und/oder schnittsicher ausgeführt ist.

12. Beutel (100, 200, 300, 400) nach einem der vorstehenden Ansprüche, **dadurch gekennzeichnet, dass** der Beutel ferner ein Absorbermaterial aufweist, mittels dessen Flüssigkeiten absorbierbar sind.

13. Beutel (100, 200, 300, 400) nach einem der vorstehenden Ansprüche, **dadurch gekennzeichnet, dass** der Beutel nach dem Verschließen gasdicht abgeschlossen ist und/oder Mittel für eine Evakuierung des Beutels vorgesehen sind.

14. Beutel (100, 200, 300, 400) nach einem der vorstehenden Ansprüche, **dadurch gekennzeichnet, dass** der Beutel ferner eine Aufhängelochung (402) und/oder eine Abrissperforation (403) aufweist.

15. Beutel (100, 200, 300, 400) nach Anspruch 3, **dadurch gekennzeichnet, dass** der Beutel beiderseits der Abrissperforation (403) identische Identifizierungsmerkmale (404, 405) aufweist, die eine Zuordnung des Abrissrests zu einem abgerissenen Beutel, insbesondere für Dokumentationszwecke, ermöglichen.
